Europäisches Patentamt

European Patent Office

Office européen des brevets

(11) Publication number: **0 299 782 B1**

# EUROPEAN PATENT SPECIFICATION

(45) Date of publication of patent specification: **07.04.93**　(51) Int. Cl.⁵: **C12N 15/00**, C12P 21/02

(21) Application number: **88306486.7**

(22) Date of filing: **15.07.88**

Consolidated with 88906635.3/0370052
(European application No./publication No.) by
decision dated 22.01.91.

The file contains technical information submitted
after the application was filed and not included in
this specification

(54) **Expression vectors for the production of human Granulocyte-Macrophage Colony Stimulation Factor in a mammalian cell host(18.05.92).**

(30) Priority: **17.07.87 US 74988**

(43) Date of publication of application:
**18.01.89 Bulletin 89/03**

(45) Publication of the grant of the patent:
**07.04.93 Bulletin 93/14**

(84) Designated Contracting States:
**AT BE CH DE ES FR GB GR IT LI LU NL SE**

(56) References cited:
**WO-A-86/00639**
**WO-A-86/03225**
**WO-A-87/02060**

**Mol. Cell. Biol. 8 (1), 466-72 (1988)**

(73) Proprietor: **Schering Biotech Corporation**
**901 California Avenue**
**Palo Alto California 94304-1104(US)**

(72) Inventor: **Yokota, Takashi**

**890 Colorado Avenue**
**Palo Alto California 94303(US)**
Inventor: **Lee, Frank D.**
**212 Rinconada Avenue**
**Palo Alto California 94301(US)**
Inventor: **Rennick, Donna M.**
**601 Almond Avenue**
**Los Altos California 94022(US)**
Inventor: **Arai, Ken-ichi**
**648 Georgia Avenue**
**Palo Alto California 94306(US)**
Inventor: **Arai, Naoko**
**648 Georgia Avenue**
**Palo Alto California 94306(US)**

(74) Representative: **Ritter, Stephen David et al**
**Mathys & Squire 10 Fleet Street**
**London EC4Y 1AY (GB)**

**Description**

Field of the Invention

The invention relates to an expression vector for the production of a protein mediator of human hematopoietic system growth and development, namely human granulocyte-macrophage colony stimulating factor (GM-CSF), in a mammalian host cell, and to a novel promoter useful in the production thereof.

BACKGROUND

Circulating blood cells are constantly replaced by newly developed cells. Replacement blood cells are formed in a process termed hematopoiesis which involves the production of at least eight mature blood cell lineages: red blood cells (erythrocytes), macrophages (monocytes), eosinophilic granulocytes, megakaryocytes (platelets), neutrophilic granulocytes, basophilic granulocytes (mast cells), T lymphocytes, and B lymphocytes (Burgess and Nicola, Growth Factors and Stem Cells (Academic Press, New York, 1983)). Much of the control of blood cell formation is mediated by a group of interacting glycoproteins termed colony stimulating factors (CSFs). These glycoproteins are so named because of the in vivo and in vitro assays used to detect their presence. Techniques for the clonal culture of hematopoietic cells in semisolid culture medium have been especially important in the development of in vitro assays. In such cultures, individual progenitor cells (i.e., cells developmentally committed to a particular lineage, but still capable of proliferation) are able to proliferate to form a colony of maturing progeny in a manner which is believed to be essentially identical to the comparable process in vivo. The role of CSFs in hematopoiesis is the subject of many recent reviews, e.g. Metcalf, The Hemopoietic Colony Stimulating Factors (Elsevier, New York, 1984); Metcalf, Science, Vol. 229, pgs. 16-22 (1985); Nicola et al., Immunology Today, Vol. 5, pgs. 76-80 (1984); Whetton et al., TIBS, Vol. 11, pgs. 207-211 (1986); and Clark and Kamen, Science, Vol. 236, pgs. 1229-1237 (1987).

The detection, isolation and purification of these factors is frequently extremely difficult, owing to the complexity of the supernatants they are typically located in, the divergencies and cross-overs of activities of the various components in the mixtures, the sensitivity (or lack thereof) of the assays utilized to ascertain the factors' properties, the frequent similarity in the range of molecular weights and other characteristics of the factors, and the very low concentration of the factors in their natural settings.

As more CSFs become available, primarily through molecular cloning, interest has heightened in finding clinical applications for them. Because of physiological similarities to hormones (e.g., soluble factors, growth mediators, action via cell receptors), potential uses of CSFs have been analogized to the current uses of hormones; e.g. Dexter, Nature, Vol. 321, pg. 198 (1986). Their use has been suggested for several clinical situations where the stimulation of blood cell generation would be desirable, such as for rehabilitative therapy after chemotherapy or radiation therapy of tumors, treatment of myeloid hypoplasias, treatment of neutrophil deficiency, treatment to enhance hematopoietic regeneration following bone marrow transplantation, and treatment to increase host resistance to established infections; e.g. Dexter (cited above), Metcalf, Science (cited above), and Clark and Kamen (cited above).

Non-recombinant GM-CSF has been purified from culture supernatants of the Mo cell line (described in U.S. Patent 4,438,032), and the first sixteen amino acids from the N-terminus have been sequenced (Gasson et al., Science, Vol. 226, pgs. 1339-1342 (1984)). Complementary DNAs (cDNAs) for GM-CSF, a factor which supports growth and development of granulocytes and macrophages, have recently been cloned and sequenced by a number of laboratories for several species; see e.g. Gough et al., Nature, Vol. 309, pgs. 763-767 (1984) (mouse); Lee et al., Proc. Natl. Acad. Sci., Vol. 82, pgs. 4360-4364 (1985) (human); Wong et al., Science, Vol. 228, pgs. 810-815 (1985) (human and gibbon); and Cantrell et al., Proc. Natl. Acad. Sci., Vol. 82, pgs. 6250-6254 (1985) (human).

A frequently-used promoter in plasmids carrying DNA sequences encoding lymphokines, e.g. human GM-CSF, has been the SV40 promoter.

SUMMARY OF THE INVENTION

The present invention is directed to expression and/or cloning vectors for GM-CSF comprising a novel promoter, designated herein the "SRα" promoter, useful in the production of human granulocyte-macrophage colony stimulating factor (GM-CSF). In particular, the invention includes the following covalently linked elements in sequence (as discussed in more detail below): SV40 origin of DNA replication (SV40 ori), an SV40 early region Promoter (SV40 early) SRα promoter, splice junction, and a GM-CSF coding region

2

(in either order), and a polyadenylation site (polyA site).

## DETAILED DESCRIPTION OF THE INVENTION

The invention therefore provides an expression vector for the production of human granulocyte-macrophage colony stimulating factor in a mammalian cell host, the expression vector comprising in sequence:

an SV40 origin of DNA replication;

an SV40 early region promoter;

a promoter;

a splice junction and a nucleotide sequence capable of encoding human granulocyte-macrophage colony stimulating factor; and

a polyadenylation site;

characterized in that the promoter is the SRα promoter of the plasmid deposited in ATCC 67318.

This expression vector preferably further includes in sequence after said SV40 polyadenylation site:

a bacterial origin of replication permitting said expression vector to be cloned in a bacterial host; and

a selectable marker permitting the identification of bacterial hosts transformed by said expression vector.

The human GM-CSF produced by an expression vector according to the invention may be glycosylated or unglycosylated according to the nature of the host and its ability to effect glycosylation.

## BRIEF DESCRIPTION OF THE FIGURES

Figure 1 illustrates the nucleotide sequence and corresponding amino acid sequence of a cDNA insert which encodes a polypeptide exhibiting human GM-CSF activity.

Figure 2A illustrates pcD-human-GM-CSF, a plasmid carrying a cDNA insert which encodes a polypeptide exhibiting human GM-CSF activity.

Figure 2B is a restriction endonuclease cleavage map of the cDNA insert of Figure 2A.

Figure 3 diagrammatically illustrates restriction sites and major coding regions of plasmid pL1.

Figure 4 diagrammatically illustrates restriction sites and major coding regions of plasmid pcDV1.

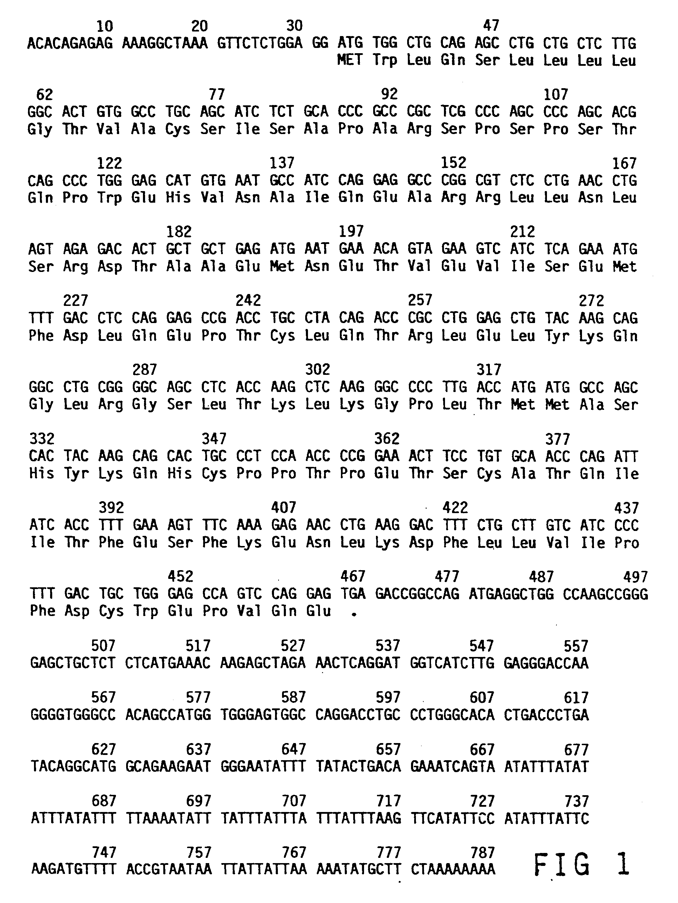

The clone pcD-human-GM-CSF illustrated in Figure 1 has been deposited with the American Type Culture Collection, Rockville, Maryland, under accession number 39923.

The expresssion or cloning vector according to the invention provides the following elements in sequence:

SV40 ori

SV40 early promoter

SRα promoter

splice junction

GM-CSF coding region

poly A site

[vector ring closes onto SV40 ori, above)

Preferably, the expression and/or cloning vector further includes, inserted between the SV40 ori and the polyA site, a bacterial origin of replication (bacterial ori) and a gene for a selectable marker (as illustrated below):

SV40 ori

SV40 early promoter

SRα promoter

splice junction

GM-CSF coding region

poly A site

bacterial ori

selectable marker

[vector ring closes onto SV40 ori, above

More preferably, the gene for the selectable marker confers drug resistance to a host bacterium, such as resistance to neomycin, ampicillin, tetracycline, streptomycin, kanamycin, hygromycin, or the like. Most preferably, the bacterial ori is the pBR322 ori.

Throughout, standard abbreviations are used to designate amino acids, nucleotides, restriction endonucleases, and the like; e.g. Cohn, "Nomenclature and Symbolism of α-Amino Acids, "Methods in Enzymology, Vol. 106, pgs. 3-17 (1984); Wood et al. Biochemistry: A Problems Approach, 2nd ed.

(Benjamin, Menlo Park, 1981); and Roberts, "Directory of Restriction Endonucleases", Methods in Enzymology, Vol. 68, pgs. 27-40 (1979).

The human GM-CSF produced by the expression vectors according to the present invention is capable of stimulating the regeneration of blood cells, and may be useful in cancer therapy, the treatment of certain blood diseases, and the treatment of persistent infections.

GM-CSF produced according to the present invention includes glycosylated or unglycosylated polypeptides which exhibit human GM-CSF activity. The invention also includes methods of making the glycosylated or unglycosylated polypeptides of the invention which utilize the SRα promoter disclosed herein.

Techniques for making, using, and identifying the human GM-CSF produced according to the invention and discussed below in general terms. Afterwards several specific examples are provided wherein the general techniques are applied using specific cell types, vectors, reagents, and the like.

## I. De Novo Preparation of GM-CSF cDNA

A variety of methods are now available for de novo preparation and cloning of cDNAs and for the construction of cDNA libraries. A review of relevant literature, together with procedural details, is given at page 29, line 22 to page 32, line 5 from the bottom of Application PCT/US 86/02464, published as WO 87/02990. A preferred source of mRNA encoding the polypeptides of the present invention is discussed in the next paragraph. The disclosure in Application PCT/US 86/02464 must be read here in relation to the production of GM-CSF, especially on page 31 thereof.

A preferred source of mRNA encoding the desired polypeptides is cells whose supernatants contain one of the activities associated with the polypeptides of the present invention, such as T lymphocytes. RNAs for deriving GM-CSF cDNAs can be obtained from the Mo cell line disclosed in U.S. Patent 4,438,032 and deposited in the American Type Culture Collection under accession number CRL 8066. In general, suitable T cells can be obtained from a variety of sources, such as human spleen, tonsils and peripheral blood. T cell clones, such as those isolated from peripheral blood T-lymphocytes, may also be used (see Research Monographs in Immunology, eds. von Doehmer and Haaf, Section D: "Human T Cell Clones", Vol. 8, pgs. 243-333; Elsevier Science Publishers, N.Y. (1985)).

## II. Preparation of GM-CSF cDNAs via Hybridization Probes Derived from Disclosed cDNA

Very often within a population several closely related forms of a protein exist which perform the same biological function. The best studied examples are (1) the so-called "allozymes", which are allelic forms of an enzyme that can be distinguished by electrophoresis; see e.g. Sensabaugh, "The Utilization of Polymorphic Enzymes in Forensic Science," Isozymes, Vol. 11, pgs. 137-154 (1983), and Lewontin, chapter 3 in The Genetic Basis of Evolutionary Change (Columbia University Press, New York, (1974); (2) the so-called "allotypes", which are polymorphisms of the constant regions of immunoglobulins, e.g. Hood et al., Immunology, pgs. 231-238 (Benjamin/Cummings, Menlo Park, 1978); and (3) hemoglobins, e.g. Dickerson and Geis, Hemoglobin (Benjamin/Cummings, Menlo Park, 1983). Such polymorphisms are also believed to exist among lymphokines: (i) two forms of human GM-CSF have been reported in PCT Patent Application WO 86/00639; and (ii) Wong et al. in Science, Vol. 235, pgs. 1504-1508, report a separate form of human CSF-1 from that reported by Kawasaki et al. in Science, Vol. 230, pgs. 291-296 (1985).

## III. Assays for GM-CSF Activity

To determine GM-CSF activity, hemopoietic cells, e.g. bone marrow cells or fetal cord blood cells are made into a single cell suspension. The individual cells are then "immobilized" in a semi-solid (agar) or viscous (methylcellulose) medium containing nutrients and usually fetal calf serum. In the presence of an appropriate stimulating factor, individual cells will proliferate and differentiate. Since the initial cells are immobilized, colonies develop as the cells proliferate and mature. These colonies can be scored after 7-14 days. Detailed instructions for conducting GM-CSF assays are given by Burgess, A., Growth Factors and Stem Cells, pgs. 52-55 (Academic Press, New York 1984), and Metcalf, The Hemopoietic Colony Stimulating Factors, pgs. 103-125 (Elsevier, New York, 1984), both sections of these references being incorporated by reference. If desired, individual colonies can be extracted, placed on microscope slides, fixed and stained with Wright/Geimsa (Todd-Sanford, Clinical Diagnosis by Laboratory Methods, 15th Edition, Eds. Davidson and Henry [1974]). Morphological analysis of cell types present per single colony can then be determined.

Bone marrow cells collected from patients with nonhematologic disease are layered over Ficoll (type 400, Sigma Chemical Co., St. Louis, MO), centrifuged (600 x g, 20 min), and the cells at the interface removed. These cells are washed twice in Iscove's Modified Dulbecco's Medium containing 10% fetal calf serum (FCS) and resuspended in the same medium, and the adherent cells removed by adherence to plastic Petri dishes (adherent cells are frequently GM-CSF producing cells: Metcalf (cited above)). The nonadherent cells are added at $10^5$ cells/ml to Iscove's Medium containing 20% FCS, 50 $\mu$M 2-mercaptoethanol, 0.9% methylcellulose and various concentrations of either supernatants known to contain colony stimulating activity or test supernatants. One ml aliquots are plated in 35 mm petri dishes and cultured at 37°C in a fully humidified atmosphere of 6% $CO_2$ in air. Three days after the initiation of the culture, 1 unit of erythropoietin is added to each plate. Granulocyte-macrophage colonies and erythroid bursts are scored at 10-14 days using an inverted microscope.

Cord blood cells collected in heparin are spun at 600 x g for 6 min. The white blood cells at the interface between the plasma and red blood cell peak are transferred to a tube containing 0.17 N ammonium chloride and 6% FCS. After 5 min on ice, the suspension is underlaid with 4 ml FCS and centrifuged for 6 minutes at 600 x g. The cell pellet is washed with Dulbecco's phosphate buffered saline and put through the Ficoll and plastic adherence steps as described above for bone marrow cells. The low-density nonadherent cells are collected and placed at $10^5$ cells/culture in the semi-solid culture medium as described above.

At the end of the assays, the cellular composition is determined after applying the individual colonies to glass slides and staining with Wright-Geimsa. Eosinophils are determined by staining with Luxol Fast Blue, e.g. Johnson, G. and Metcalf, D., Exp. Hematol., Vol. 8, pgs. 549-561 (1980).

## IV. Purification and Pharmaceutical Compositions

The human GM-CSF of the present invention expressed in E. coli, in yeast or in other cells can be purified according to standard procedures of the art, including ammonium sulfate precipitation, fractionation column chromatography (e.g., ion exchange, gel filtration, electrophoresis, affinity chromatography, etc.) and ultimately crystallization (see generally "Enzyme Purification and Related Techniques," Methods in Enzymology, 22:233-577 [1977] and Scopes, R., Protein Purification: Principles and Practice, Springer-Verlag, New York [1982]). The precise purification protocol selected depends, to a large extent, on the expression host used. For example, mammalian expression hosts frequently require the presence of serum in their growth medium, which necessitates additional steps for removing the serum proteins. Yeast and bacteria, on the other hand, grow on defined media from which it is usually simpler to separate secreted products. Some expression hosts may not secrete the expressed product, and then the product must be separated from a homogenate or extract of expression host. The purification problems encountered in all of these situations are readily solvable by those with ordinary skill in the art of biochemical purifications.

Once purified, partially or to homogeneity, the human GM-CSF of the invention may be used for research purposes, e.g., as a supplement to cell growth media (e.g., minimum essential medium Eagle, Iscove's modified Dulbecco Medium or RPMI 1640, available from Sigma Chemical Company (St. Louis, MO) and GIBCO Division (Chagrin Falls, OH)) and as an antigenic substance for eliciting specific immunoglobulins useful in immunoassays, immunofluorescent stainings, etc. (See generally Immunological Methods, Vols. I & II, Eds. Lefkovits, I. and Pernis, B., Academic Press, New York, N.Y. [1979 & 1981], and Handbook of Experimental Immunology, ed. Weir, D., Blackwell Scientific Publications, St. Louis, MO [1978].)

The human GM-CSF of the present invention may also be used in pharmaceutical compositions, e.g., to enhance natural defenses against persistent infections or to promote blood cell regeneration. Thus, patients with rheumatoid arthritis, in need of a transplant, or with immunodeficiency caused by cancer chemotherapy, advanced age, immunosuppressive agents, etc., may be treated directly with such polypeptides. Or populations of hematopoietic cells of one person may be maintained and/or expanded or caused to differentiate ex vivo for eventual reintroduction into the same or another person for a beneficial effect. The compositions can selectively stimulate various components of the immune system, either alone or with other agents well known to those skilled in the art. In particular, the compositions may include other immune-reactive agents, such as lymphokines (e.g. IL-1, IL-2, IL-3, IL-4, G-CSF, M-CSF, or the like).

Pharmaceutical compositions containing the human GM-CSF of this invention can be prepared and used as described in Application PCT/US 86/02464 at page 44, line 27 to page 45, line 21. In particular, the quantity of active compound in a unit dose of preparation may be varied or adjusted from 1 $\mu$g to 100 mg, according to the particular application and the potency of the active ingredient.

## V. Expression Systems

Once the cDNA of the invention has been cloned, a wide range of expression systems (i.e. host-expression vector combinations) can be used to produce the proteins of the invention. Possible types of host cells include, but are not limited to, bacterial, yeast, insect, mammalian, and the like. Selecting an expression system, and optimizing protein production thereby, requires the consideration and balancing of many factors, including (1) the nature of the protein to be expressed, e.g. the protein may be poisonous to some host organisms, it may be susceptible to degradation by host proteases, or it may be expressed in inactive conformations or in insoluble form in some hosts, (2) the nature of the messenger RNA (mRNA) corresponding to the protein of interest, e.g. the mRNA may have sequences particularly susceptible to host endonucleases, which drastically reduce the functional lifetime of the mRNA, or the mRNA may form secondary structures that mask the start codon or ribosome binding site, thereby inhibiting translation initiation in some hosts, (3) the selection, availability, and arrangement of host-compatible expression control sequences in the 3'- and 5'-regions flanking the coding region -- these include promoters, 5'- and 3'-protector sequences, ribosome binding sites, transcription terminators, enhancers, polyadenylate addition sites, cap sites, intron-splice sites, and the like, (4) whether the protein has a secretion signal sequence which can be processed by the host, or whether an expression control sequence encoding a signal sequence endogenous to the host must be spliced onto the region encoding the mature protein, (5) the available modes and efficiencies of transfection or transformation of the host, and whether transient or stable expression is desired, (6) the scale and cost of the host culture system desired for expressing the protein, (7) whether, and what type of, posttranslational modifications are desired, e.g. the extent and kind of glycosylation desired may affect the choice of host, e.g. Uy and Wold, Science, Vol. 198, pgs. 890-896 (1977), (8) the ease with which the expressed protein can be separated from proteins and other materials of the host cells and/or culture medium e.g. in some cases it may be desirable to express a fusion protein with a specialized signal sequence to aid in later purification steps, (9) the stability and copy number of a particular vector in a selected host, e.g. Hofschneider et al., eds. Gene Cloning in Organisms Other than E. Coli (Springer Verlag, Berlin, 1982), and (10) like factors known to those skilled in the art.

Many reviews are available which provide guidance for making choices and/or modifications of specific expression systems in light of the recited factors: e.g., de Boer and Shepard, "Strategies for Optimizing Foreign Gene Expression in Escherichia coli," pgs. 205-247, in Kroon, ed. Genes: Structure and Expression (John Wiley & Sons, New York, 1983), review several E. coli expression systems; Kucherlapati et al., Critical Reviews in Biochemistry, Vol. 16, Issue 4, pgs. 349-379 (1984), and Banerji et al., Genetic Engineering, Vol. 5, pgs. 19-31 (1983) review methods for transfecting and transforming mammalian cells; Reznikoff and Gold, eds. Maximizing Gene Expression (Butterworths, Boston, 1986) review selected topics in gene expression in E. coli, yeast, and mammalian cells; and Thilly Mammalian Cell Technology (Butterworths, Boston, 1986) reviews mammalian expression systems.

Likewise, many reviews are available which describe techniques and conditions for linking and/or manipulating specific cDNAs and expression control sequences to create and/or modify expression vectors suitable for use with the present invention: e.g. Maniatis et al., Molecular Cloning: A Laboratory Manual - (Cold Spring Harbor Laboratory, N.Y., 1982); Glover, DNA Cloning: A Practical Approach, Vol. I and II (IRL Press, Oxford, 1985); and Perbal, A Practical Guide to Molecular Cloning (John Wiley & Sons, N.Y., 1984). Generally, within an expression vector various sites may be selected for insertion of the cDNA of the invention. These sites are usually designated by the restriction endonuclease which cuts them and are well recognized by those of skill in the art. Various methods for inserting DNA sequences into these sites to form recombinant DNA molecules are also well known. These include, for example, dG-dC or dA-dT tailing, direct ligation, synthetic linkers, exonuclease and polymerase-linked repair reactions followed by ligation, or extension of the DNA strand with DNA polymerase and an appropriate single-stranded template followed by ligation.

Often a vector containing the cDNA of the invention must be obtained in large quantities before transfection and/or transformation of cells in a host culture can take place. For this purpose the vector is often replicated without significant expression in an organism (the cloning host) other than the one finally used for expression. Then, after propagation, the vectors are separated from the cloning host using standard techniques, e.g. as disclosed by Maniatis et al. (cited above).

"Digestion" of DNA refers to catalytic cleavage of the DNA usually with an enzyme that acts only at certain locations in the DNA. For the most part such enzymes are restriction endonucleases, and the sites on the DNA for which each is specific are called a restriction site. The various restriction enzymes used herein are commercially available and their reaction conditions, cofactors, and other requirements as established by the enzyme suppliers are used. Generally, about 1 microgram of plasmid or DNA fragment is used with about 1 unit of enzyme in about 20 microliters of buffer solution. Appropriate buffer and substrate amounts for particular restriction enzymes are specified by the manufacturer. Incubation times of about 1 hour at 37°C are ordinarily used, but may vary in accordance with the supplier's instructions. After incubation, protein is removed by extraction with phenol and chloroform, and the digested nucleic acid is recovered from the aqueous fraction by precipitation with ethanol.

"Isolation" or "purification" of a given DNA fragment from a restriction (or other) digest means separation of the digest by polyacrylamide gel electrophoresis, identification of the fragment of interest by comparison of its mobility versus that of marker DNA fragments of known molecular weight, removal of the gel section containing the desired fragment, and separation of the gel from DNA. Isolation procedures are well known, e.g. Lawn et al., Nucleic Acids Research, Vol. 9, pgs. 6103-6114 (1981), Goeddell et al. Nucleic Acids Research, Vol. 8, pg. 4057 (1980), and Maniatis et al. (cited above).

"Ligation" refers to the process of forming phosphodiester bonds between two double stranded nucleic acid fragments. Ligation may be accomplished using known buffers and conditions with 10 units of T4 DNA ligase per 0.5 micrograms of approximately equimolar amounts of the DNA fragments to be ligated.

"Amplification" of a plasmid means transforming a suitable cloning host and propagating the host to increase the total number of plasmids.

"Kinased" DNA fragments refer to fragments which have been phosphorylated with polynucleotide kinase. Such treatment enhances the efficiency of ligation of DNA fragments lacking 5'-phosphates.

Suitable prokaryote expression vectors include plasmids from E. coli, e.g. Col E1, pCR1, pBR322, pMB9 and their derivatives, wider host range plasmids, e.g. RP4, phage DNAs, such as phage lambda and its various derivatives, M13, and the like. Additional E. coli vectors are described in Chapter 12 of Maniatis et al. (cited above) for expressing eukaryotic proteins, either fused or unfused with prokaryotic peptides. And Riggs discloses still further E. coli expression systems in U.S. Patent 4,431,739, which is incorporated by reference.

Commonly used prokaryotic promoters include the $\beta$-lactamase (penicillinase) and lactose promoter systems (Chang et al., Nature, Vol. 275, pg. 615 (1978); Itakura, et al., Science, Vol. 198, pg. 1056 (1977); Goeddel, et al. Nature Vol. 281, pg. 544 (1979)); and the tryptophan (trp) promoter system (Goeddel, et al., Nucleic Acids Res., Vol. 8, pg. 4057 (1980); EPO Appl Publ No. 0036776). Whereas these are the most commonly used, other microbial promoters have been discovered and utilized, and details concerning their nucleotide sequences have been published, enabling a skilled worker to ligate them functionally with plasmid vectors, e.g. Siebenlist et al., Cell, Vol. 20, pg. 269 (1980).

A particularly useful prokaryotic promoter for high expression in E. coli is the tac promoter, disclosed by de Boer in U.S. Patent 4,551,433, which is incorporated herein by reference. Secretion expression vectors are also available for E. coli hosts. Particularly useful are the pIN-III-ompA vectors, disclosed by Ghrayeb et al. in EMBO J., Vol. 3, pgs. 2437-2442 (1984), in which the cDNA to be transcribed is fused to the portion of the E. coli ompA gene encoding the signal peptide of the ompA protein which, in turn, causes the mature protein to be secreted into the periplasmic space of the bacteria. Likewise, U.S. Patents 4,336,336 and 4,338,397 disclose secretion expression vectors for prokaryotes. Accordingly, these references are incorporated by reference.

Numerous strains of bacteria are suitable hosts for prokaryotic expression vectors including strains of E. coli, such as W3110 (ATCC No. 27325), JA221, C600, ED767, DH1, LE392, HB101, X1776 (ATCC No. 31244), X2282, RR1 (ATCC No. 31343) MRCl; strains of Bacillus subtilus; and other enterobacteriaceae such as Salmonella typhimurium or Serratia marcescens, and various species of Pseudomonas. General methods for deriving bacterial strains, such as E. coli K12 X1776, useful in the expression of eukaryotic proteins is disclosed by Curtis in U.S. Patent 4,190,495. Accordingly this patent is incorporated by reference.

Eukaryotic microbes, such as yeast cultures, can also be used to express proteins of the invention. Saccharomyces cerevisiae, or common baker's yeast, is the most commonly used among eukaryotic microorganisms, although a number of other strains are commonly available. For expression in Saccharomyces, the plasmid YRp7 can be used, e.g. Stinchcomb, et al, Nature, Vol. 282, pg. 39 (1979), Kingsman et al, Gene, Vol. 7, pg. 141 (1979), and Tschemper, et al., Gene, Vol. 10, pg. 157 (1980). This plasmid already contains the trp1 gene which provides a selection marker for a mutant strain of yeast lacking the ability to grow in tryptophan, for example ATCC No. 44076 or PEP4-1 (Jones, Genetics, Vol. 85, pg. 12 (1977)). The presence of the trp1 lesion as a characteristic of the yeast host cell genome then provides an effective environment for detecting transformation by growth in the absence of tryptophan. Additional vectors for yeast include the pGAL plasmids disclosed by Miyajima et al., Nucleic Acids Research, Vol. 12, pgs. 6397-6414 (1984), and the 2$\mu$m plasmid disclosed by Beggs, Nature, Vol. 275, pgs. 104-109 (1978).

Suitable promoting sequences in yeast vectors include the promoters for 3-phosphoglycerate kinase (Hitzeman, et al., J. Biol. Chem., Vol. 255, pg. 2073 (1980)) or other glycolytic enzymes (Hess, et al, J. Adv. Enzyme Reg., Vol. 7, pg. 149 (1968); Holland, et al, Biochemistry, Vol. 17, pg. 4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triose-phosphate isomerase, phosphoglucose isomerase, and glucokinase. In constructing suitable expression plasmids, the termination sequences associated with these genes are also ligated into the expression vector adjacent to the 3'-end of the sequence to be expressed to provide polyadenylation and termination. Other promoters, which have the additional advantage of transcription controlled by growth conditions, are the promoter regions for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization (Holland, cited above). Yet another regulatable promoter is the metallothionein promoter system disclosed by Fogel et al., in U.S. Patent 4,511,652, incorporated herein by reference. Virtually any plasmid vector containing a promoter, origin of replication and termination sequences that are all yeast-compatible is suitable.

Secretion expression vectors are also available for S. cerevisiae hosts, e.g. pMFα8 disclosed by Miyajima et al., Gene, Vol. 37, pgs. 155-161 (1985); YEpIPT disclosed by Hitzeman et al., Science, Vol. 219, pgs. 620-625 (1983); pYαEGF-21 disclosed by Brake et al., Proc. Natl. Acad. Sci., Vol. 81, pgs. 4642-4646 (1984) and by Brake in European Patent Application 0116201; and plasmids disclosed by Singh in European Patent Application 0123544 and by Kurjan et al. in U.S. Patent 4,546,082.

In addition to prokaryotic and eukaryotic microorganisms, expression systems comprising cells derived from multicellular organism may also be used to produce proteins of the invention. Of particular interest are mammalian expression systems because their posttranslational processing machinery is more likely to produce biologically active mammalian proteins. Several DNA tumor viruses have been used as vectors for mammalian hosts: e.g. see Tooze, ed., DNA Tumor Viruses, 2nd Ed. (Cold Spring Harbor Laboratory, N.Y., 1981) for a review of their biology. Particularly important are the numerous vectors which comprise SV40 replication, transcription, and/or translation control sequences coupled to bacterial replication control sequences, e.g. the pcD vectors developed by Okayama and Berg, disclosed in Mol. Cell. Biol., Vol. 2, pgs. 161-170 (1982) and Mol. Cell. Biol., Vol. 3, pgs. 280-289 (1983), both of which are incorporated herein by reference; the SV40 vectors disclosed by Hamer in Genetic Engineering, Vol. 2, pgs. 83-100 (1980), and U.S. Patent 4,599,308, both of which are incorporated herein by reference; and the vectors additionally containing adenovirus regulatory elements, disclosed by Kaufman and Sharp, in Mol. Cell. Biol., Vol. 2, pgs. 1304-1319 (1982), and Kaufman et al., in European Patent Application 8406107, both of which are incorporated herein by reference. Monkey cells are usually the preferred hosts for the above vectors. Such vectors containing the SV40 ori sequences and an intact A gene can replicate autonomously in monkey cells (to give higher copy numbers and/or more stable copy numbers than nonautonomously replicating plasmids). Moreover, vectors that contain the SV40 ori sequences without an intact A gene and can replicate autonomously to high copy numbers (but not stably) in COS7 monkey cells are described by Gluzman, Cell, Vol. 23, pgs. 175-182 (1981) and are available from the ATCC (accession no. CRL 1651). The above SV40-based vectors are also capable of transforming other mammalian cells, such as mouse L cells, by integration into the host cell DNA.

Besides the SV40-based vectors, mammalian expression systems suitable for use with the present invention include, but are not limited to (i) vectors comprising bovine papilloma virus (BPV) sequences, e.g. BPV-pBR322 hybrids disclosed by Sarver et al., Genetic Engineering, Vol. 5, pgs. 173-190 (1983), and DiMaio et al., Proc. Natl. Acad. Sci., Vol. 79, pgs. 4030-4034 (1982), for transforming bovine and mouse cells; (ii) vectors comprising Epstein-Barr virus (EBV) sequences, e.g. plasmids carrying the EBV oriP sequences (including the coding sequences for the nuclear antigen EBNA-1) disclosed by Yates et al., Nature, Vol. 313, pgs. 812-815 (1985), Reisman et al., Mol. Cell. Biol., Vol. 5, pgs. 1822-1832 (1985), Yates et al., Proc. Natl. Acad. Sci., Vol. 81, pgs. 3806-3810 (1984), and Sugden et al., Mol. Cell. Biol., Vol. 5, pgs. 410-413 (1985), for stable transformation of various mammalian cells including human and monkey cells; (iii) vectors comprising murine polyoma virus sequences, e.g. O'Hara, J. Mol. Biol., Vol. 151, pg. 203 (1981), for transforming mouse and hamster cells; (iv) vectors carrying the dihydrofolate reductase (dhfr) gene, e.g. Alt et al., J. Biol. Chem., Vol. 253, pgs. 1357-1370 (1978), which in response to methotrexate treatment duplicates together with adjacent coding regions cointegrated into murine genomes (e.g., that of a Chinese hamster ovary (CHO) cell line deficient in dhfr activity) e.g. as described by Urlamb et al., Proc. Natl. Acad. Sci., Vol. 77, pg. 4216 (1980); and (v) cotransformation systems such as that disclosed by Axel et al. in U.S. Patent 4,399,216. Additional mammalian expression vectors can be constructed, or existing ones modified, by using various elements from available DNA tumor viruses and retroviruses, e.g. origins of replication, enhancer sequences (such as the long terminal repeat sequence from Rous sarcoma virus (RSV-LTR) disclosed by Gorman et al., Proc. Natl. Acad. Sci., Vol. 79, pgs. 6777-6781 (1982)) intron-splice sites, polyadenylation sites, and the like.

Invertebrate expression systems can also be constructed for use with the invention, e.g. the larvae of silk worm, Bombyx mori, infected by a baculovirus vector, BmNPV, described by Maeda et al. in Nature, Vol. 315, pgs. 892-894 (1985), and in Saibo Koguku, Vol. 4, pgs. 767-779 (1985).

EXAMPLES

The following examples serve to illustrate the present invention. Selection of cDNA libraries, vectors, and hosts as well as the concentration of reagents, temperatures, and the values of other variables are only to exemplify application of the present invention and are not to be considered limitations thereof.

The Examples describe the production and purification of mature GM-CSF (127 aminoacids long).

Example I. Construction of cDNA Libraries from Peripheral Blood Lymphocytes and T Cell Clones, Isolation of cDNA Clones, and Expression in COS7 Monkey Cells

cDNA libraries were constructed from mRNA isolated from a human clonal T cell line, designated T-7, and from human peripheral blood lymphocytes (PBLs). The libraries were constructed in the pcD plasmid according to the method of Okayama and Berg (discussed above). After a single clone was isolated from the T-7 library, the existence of an identical clone in the PBL library was confirmed.

A. Cloned Helper T Cells.

A human T cell clone designated T-7 was isolated according to the method described in Chapters 36 and 37 of Isolation, Characterization, and Utilization of T Lymphocyte Clones, Eds. Fathman and Fitch, Academic Press, New York (1982). The cell line was continuously maintained at about $0.5 \times 10^5$ cells/ml in Dulbecco's Modified Eagle (DME) medium with 10% heat-inactivated fetal calf serum, $5 \times 10^{-5}$ M 2-ME, 2 mM glutamine, non-essential amino acids, and essential vitamins conditioned with 30% supernatants from phytohemagglutinin (PHA) stimulated human peripheral leukocytes.

B. Induction of GM-CSF Production.

T-7 cells were cultured at $5 \times 10^5$/ml in DME with 4% heat-inactivated fetal calf serum, $5 \times 10^{-5}$ M 2-ME, 2mM glutamine, non-essential amino acids, essential vitamins and 4 $\mu$g/ml concanavalin A (ConA). After 4-6 hours' incubation at 37°C in 10% $CO_2$, the cell suspension was centrifuged at 1500 rpm for 10 minutes. The cell pellets were collected and frozen immediately at -70°C. The supernatants were filtered (Nalgene-0.22 microns) and stored at -80°C as a source of growth factors. Aliquots of the supernatant were assayed for CSF activity (see below) to verify the induction of the line by the ConA treatment.

PBLs were induced under similar conditions, except that 7 $\mu$g/ml ConA was used.

C. Isolation of mRNA.

Total cellular RNA was isolated from cells using the guanidine isothiocyanate procedure of Chirgwin, J. et al., (Biochemistry, 18:5294-5299 [1979]). Frozen cell pellets from ConA-induced T-7 cells or PBLs (4 hrs after stimulation) were suspended in guanidine isothiocyanate lysis solution. Twenty ml of lysis solution was used for $1.5 \times 10^8$ cells. Pellets were resuspended by pipetting, then DNA was sheared by 4 passes through a 16 gauge needle using a syringe. The lysate was layered on top of 20 ml of 5.7 M CsCl, 10 mM EDTA in 40 ml polyallomer centrifuge tube. This solution was centrifuged at 25,000 rpm in Beckman SW28 rotor (Beckman Instruments, Inc., Palo Alto, CA) for 40 hrs at 15°C. The guanidine isothiocyanate phase containing DNA was pipetted off from the top, down to the interface. The walls of the tube and interface were washed with 2-3 ml of guanidine isothiocyanate lysis solution. The tube was cut below the interface with scissors, and the CsCl solution was decanted. RNA pellets were washed twice with cold 70% ethanol. Pellets were then resuspended in 500 $\mu$l of 10 mM Tris.HCl pH 7.4, 1 mM EDTA, 0.05% SDS. 50 $\mu$l of 3M sodium acetate was added and RNA was precipitated with 1 ml ethanol. About 0.3 mg total RNA was collected by centrifuging and the pellets washed once with cold ethanol.

Washed and dried total RNA pellet was resuspended in 900 $\mu$l of oligo (dT) elution buffer (10 mM Tris.HCl, pH 7.4, 1 mM EDTA, 0.5% SDS). RNA was heated for 3 min. at 68°C and then chilled on ice. 100

$\mu$l of 5 M NaCl was added. The RNA sample was loaded onto a 1.0 ml oligo (dT) cellulose column (Type 3, Collaborative Research, Waltham, MA) equilibrated with binding buffer (10 mM Tris.HCl pH 7.4, 1 mM EDTA, 0.5 M NaCl, 0.5% SDS.) Flow-through from the column was passed over the column twice more. The column was then washed with 20 ml binding buffer. PolyA[+] mRNA was collected by washing with elution buffer. RNA usually eluted in the first 2 ml of elution buffer. RNA was precipitated with 0.1 volume 3 M sodium acetate (pH 6) and two volumes of ethanol. The RNA pellet was collected by centrifugation, washed twice with cold ethanol, and dried. The pellet was then resuspended in water. Aliquots were diluted, and absorbance at 260 nm was determined.

D. Construction of pcD cDNA Library

Parts 1) and 2) steps 1) to 5) were carried out as disclosed in PCT/US 85/02464 at pages 56 to 60, except that the pcDV1 DNA (page 57, lines 3-4) was digested with NsiI endonuclease.

Step 6 (of cDNA Library Preparation): Transformation of E. coli. Transformation was carried out using minor modifications of the procedure described by Cohen et al., Proc. Natl. Acad. Sci., Vol. 69, pgs. 2110-2114 (1972). E coli K-12 strain MC1061, described by Casabadan, M. and Cohen, S. in J. Mol. Biol., Vol. 138, pgs. 179-207 (1980), was grown to 0.5 absorbancy unit at 600 nm at 37°C in 20 ml of L-broth. The cells were collected by centrifugation, suspended in 10 ml of 10 mM Tris•HCl (pH 7.3) containing 50 mM CaCl$_2$, and centrifuged at 0°C for 5 min. The cells were resuspended in 2ml of the above buffer and incubated again at 0°C for 5 min.; then, 0.2 ml of the cell suspensions was mixed with 0.1 ml of the DNA solution from step 5 and the mixture was incubated at 0°C for 15 min. Next the cells were kept at 37°C for 2 min. and thereafter at room temperature for 10 min.; then 0.5 ml of L-broth was added, and the culture was incubated at 37°C for 30 min., mixed with 2.5 ml of L-broth soft agar at 42°C, and spread over L-broth agar containing 50 $\mu$g of ampicillin per ml. After incubation at 37°C for 12 to 24 hr., individual colonies were picked with sterile toothpicks. In all, approximately $5 \times 10^4$ independent cDNA clones were generated.

E. Screening of the Human T-cell cDNA Library by DNA Transfections

A collection of $10^4$ independent clones were picked at random from the T-cell cDNA library and propagated individually in wells of microtiter dishes containing 200 $\mu$l L broth with ampicillin at 50 $\mu$g/ml dimethyl sulfoxide at 7%. Pools containing 48 cDNA clones were prepared from the microtiter cultures. 40 such pools were grown up in 1 liter cultures of L-broth containing 100 $\mu$g/ml ampicillin. Plasmid DNA was isolated from each culture and purified by twice banding through CsCl gradients. The DNA representing each pool was transfected into COS7 monkey cells as follows.

One day prior to transfection, approximately $10^6$ COS7 monkey cells were seeded onto individual 60 mm plates in DME containing 10% fetal calf serum and 2 M glutamine. To perform the transfection, the medium was aspirated from each plate and replaced with 1.5 ml of DME containing 50 mM Tris•HCl (pH 7.4), 400 $\mu$g/ml DEAE-Dextran and 15 $\mu$g of the plasmid DNAs to be tested. The plates were incubated for four hours at 37°C, then the DNA-containing medium was removed, and the plates were washed twice with 2 ml of serum-free DME. DME containing 150 $\mu$M chloroquine was added back to the plates which were then incubated for an additional 3 hours at 37°C. The plates were washed once with DME, and then DME containing 4% fetal calf serum, 2 mM glutamine, penicillin and streptomycin was added. The cells were then incubated for 72 hours at 37°C. The growth medium was collected and assayed for GM-CSF activity as described above.

Four pools (groups 1A, 3B, 7A, and 14A) yielded human GM-CSF activity (see Table I below). Each group was then subdivided into 6 pools, each containing 8 of the original pooled clones. One subpool from each initial pool was positive in the transfection assay, with the exception of 7A which yielded two positive subpools. Each of the plasmids in four of the five subpools was transfected individually into COS7 cells. Four single clones, designated 3-8a, 7-1a, 7-4d, and 14-1e were active in producing GM-CSF activity. Restriction endonuclease analysis showed that all of these clones share essentially the same structure.

Table I presents the number of hemopoietic colonies stimulated by each transfection sample in duplicate cord blood assays. A cluster represents 20 to 50 cells, a small colony was 51 to 150 cells, and a colony was more than 150 cells.

## TABLE I

### Assay of Colony Stimulating Activity
### from Plasmid DNA Pools Transfections

| First Screening | 40 pools of 48 clones (1-20, A + B) | |
|---|---|---|
| | Mock-infected COS7: | 7 + 12 clusters |
| | Pool 1A: | 29 + 25 clusters |
| | Pool 3B: | 38 + 20 clusters |
| | Pool 7A: | 22 + 19 clusters |
| | Pool 14A: | 26 + 32 clusters |
| | All other pools: | fewer than 20 clusters |

| Second Screening | Sub-pools of 8 clones | |
|---|---|---|
| | Mock-infected COS7: | 9 + 15 clusters |
| | Sub-pool 1-5: | 56 + 54 clusters |
| | Sub-pool 3-8: | 98 + 52 small colonies |
| | Sub-pool 7-1: | 29 + 41 small colonies |
| | Sub-pool 7-4: | 100 + 93 small colonies |
| | Sub-pool 14-1: | 40 + 73 small colonies |
| | All other sub-pools: | fewer than 20 clusters |

| Third Screening | Individual clones | |
|---|---|---|
| | Clone 3 - 8a: | 120 + 127 colonies |
| | Clone 7 - 1a: | 198 + 164 colonies |
| | Clone 7 - 4a: | 176 + 160 colonies |
| | Clone 14 - 1e: | 62 + 67 colonies |

All other clones fewer than 20 clusters

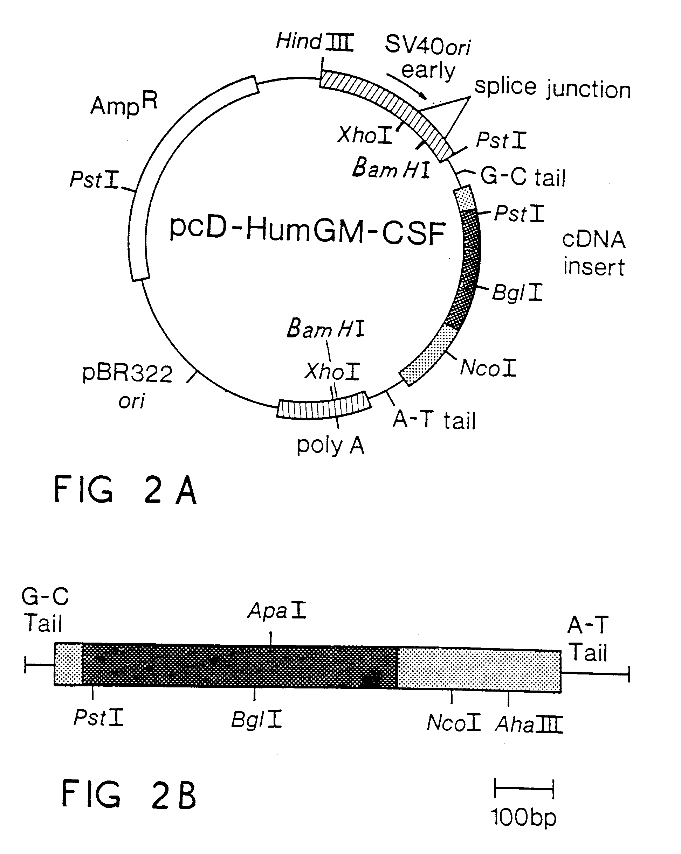

A plasmid (pcD-human-GM-CSF) carrying a substantially full-length cDNA insert is shown in Figure 2, and an E. coli bacterium (MC1061) carrying the plasmid has been deposited with the ATCC (accession number 39923). In Figure 2, transcription of the 776 bp cDNA insert contained in the pcD expression vector from the SV40 early promoter is indicated by the arrow. The locations of the splice donor and acceptor sites are shown. A polyadenylation signal derived from SV40 is located at the 3'-end of the cDNA insert.

The GM-CSF coding region in the cDNA insert is heavily shaded, while the non-coding regions are lightly shaded. The remainder of the vector sequences are derived from pBR322, including the $\beta$-lactamase gene (Amp$^R$) and the origin of replication.

Utilizing both the M13 dideoxy chain termination method (Sanger, F., et al., Proc. Natl. Acad. Sci., Vol. 74, pgs. 5463-5467 (1977) and modified Maxam/Gilbert technique (Rubin, C. and Schmid, C., Nucleic Acid Res., Vol. 8, pgs. 4613-4619 (1981) the 3-8a sequence was determined. The cDNA insert contains a single open reading frame. The first ATG is found 33-35 nucleotides from the 5'-end, and is followed by 144 codons before the termination triplet (TGA) at nucleotide positions 465-467.

Table II shows a percentage breakdown of the cellular composition of about 60 human bone marrow and cord blood colonies grown under the influence of the individual clones 3-8a, 7-1a, 7-4d, and 14-1e. The existence of mixed colonies of eosinophils and the other cell types could be due to colonies growing together.

TABLE II

| Cellular Composition of Human Bone Marrow Colonies | | | | | |
|---|---|---|---|---|---|
| Neu | M$\phi$ | Eos | Neu/M$\phi$ | M$\phi$/Eos | Neu/M$\phi$/Eos |
| 15% | 30% | 7% | 37% | 2% | 9% |

Example II. Enhanced Expression of GM-CSF in Mammalian Cells Using the SR$_\alpha$ Promoter

Enhanced expression of GM-CSF in a variety of mammalian cells is obtained by the use of a promoter (designated SR$_\alpha$) which is constructed by inserting a portion of the long terminal repeat (LTR) of an HTLV-(I) retrovirus at the Xho I site of the SV40 early origin of replication. The presence of the LTR fragment increases expression by a factor of 20-50 in COS monkey cells (e.g. available from the ATCC under accession numbers CRL 1650 or CRL1651) and CV1 monkey cells (e.g. available from the ATCC under accession number CCL 70), and in mouse L cells (e.g. L-M(TK$^-$) available from the ATCC under accession number CCL 1.3). Directions are provided below for constructing the SR$_\alpha$ promoter from synthetic DNA fragments. Alternatively, the SR$_\alpha$ promoter can be obtained from plasmid pcD-huIL-3-22-1 deposited with the ATCC under accession number 67318.

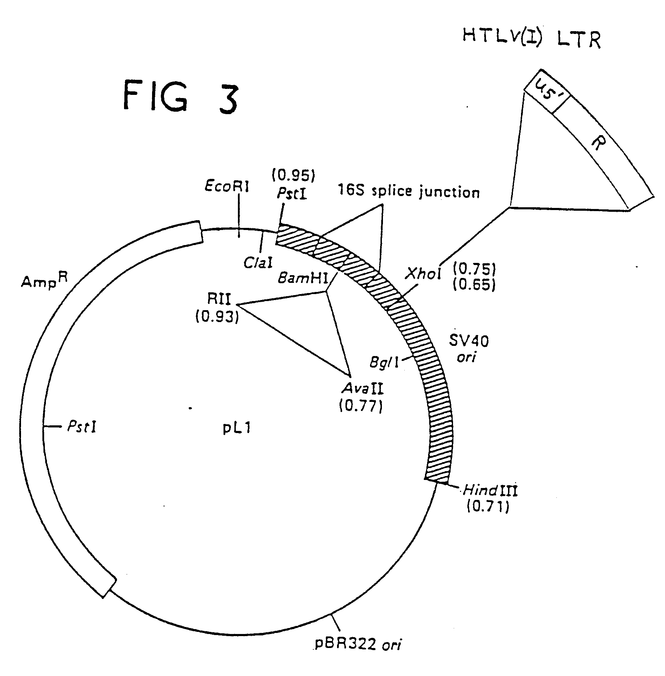

Retroviral LTRs have been shown to enhance expression in a number of systems: e.g. Chen et al., Proc. Natl. Acad. Sci., Vol. 82, pgs. 7285-7288 (1985); Gorman et al., Proc. Natl. Acad. Sci., Vol. 79, pgs. 6777-6781 (1982); Temin, Cell, Vol. 27, pgs. 1-3 (1981); and Luciw et al., Cell, Vol. 33, pgs. 705-716 (1983). The portion of the HTLV(I) LTR inserted at the XhoI site of pL1 consists of a 267 base segment including the entire R region and part of the U5 region (designated U5', in Figure 3) extending from the R/U5 boundary to the first downstream TaqI site (39 bases in all). The sequence of the HTLV(I) LTR is disclosed by Yoshida et al. in Current Topics in Microbiology and Immunology, Vol. 115, pgs. 157-175 (1985).

The HTLV(I) LTR segment is inserted into the plasmid pL1 in four steps utilizing procedures disclosed in Example IV, to form the modified plasmid, pL1', illustrated in Figure 3. First, pL1 is digested with BglI and XhoI, and the large fragment is isolated. The large fragment is then mixed with synthetics 1A/B and 2A/B (defined below) in a standard ligation mixture Synthetics 1A/B and 2A/B together consist of, in sequence, 5'-->3', the BglI/XhoI fragment of the SV40 ori, the 267-base 5'-piece of the LTR R region, and a polylinker consisting of SmaI, SacI, NaeI, and XhoI sites in the stated order.

```
(BglI)
    TCGGCCTCTGAGCTATTCCAGAAGTAGTGAGGAGGCTTTTTTGGA-
    CGGAGCCGGAGACTCGATAAGGTCTTCATCACTCCTCCGAAAAAACCT-

GGCCTAGGCTTTT
CCGG
```

Synthetics 1A/B

```
                                                            SmaI
        SV40 ori->|R----->                R----->|  SacI
            GCAAAAAGCTCGGCTCGCATCTCTCCTTCACGCGCCCGGGGAG-
    ATCCGAAAACGTTTTTCGAGCCGAGCGTAGAGAGGAAGTGCGCGGGCCCCTC-


    NaeI   XhoI
CTCGCCGGCC
GAGCGGCCGGAGCT
```

Synthetics 2A/B

After ligation, the resulting plasmid of step 1 is propagated, isolated, digested with SmaI and SacI, and the large fragment is isolated. The large fragment is then mixed with synthetics 3A/B and 4A/B (defined below in a standard ligation mixture.

```
    GCCGCCCTACCTGAGGCCGCCATCCACGCCGGTTGAGTCGCGTTCT
    CGGCGGGATGGACTCCGGCGGTAGGTGCGGCCAACTC
```

Synthetics 3A/B

```
        GCCGCCTCCCGCCTGTGGTGCCTCCTGAACTGCGTCCGC-
    AGCGCAAGACGGCGGAGGGCGGACACCACGGAGGACTTGACGCAGGCG-


    SacI
    CGTCTAGGTAAGTTTAGAGCT
    GCAGATCCATTCAAATC
```

Synthetics 4A/B

After ligation, the resulting plasmid of step 2 is propagated, isolated, digested with SacI and NaeI, and the large fragment is isolated. The large fragment is then mixed with synthetics 5A/B (defined below) in a standard ligation mixture.

```
CAGGTCGAGACCGGGCCTTTGTCCGGCGCTCCCTTGGAGCCTACCT-
TCGAGTCCAGCTCTGGCCCGGAAACAGGCCGCGAGGGAACCTCGGATGGA-
```

```
        NaeI
AGACTCAGCC
TCTGAGTCGG
```

## Synthetics 5A/B

After ligation, the resulting plasmid of step 3 is propagated, isolated, digested with NaeI and XhoI, and the large fragment is isolated. The large fragment is then mixed with synthetics 6A/B and 7A/B (defined below) in a standard ligation mixture to form the modified pL1 plasmid, designated pL1'.

```
    NaeI
        GGCTCTCCACGCTTTGCCTGACCCTGCTTGCTCAACTCTACG
        CCGAGAGGTGCGAAACGGACTGGGACGAACGAG
```

## Synthetics 6A/B

```
        XhoI
        TCTTTGTTTCGTTTTCTGTTCTGCGCCGTTACAGATC
TTGAGATGCAGAAACAAAGCAAAAGACAAGACGCGGCAATGTCTAGAGCT
```

## Synthetics 7A/B

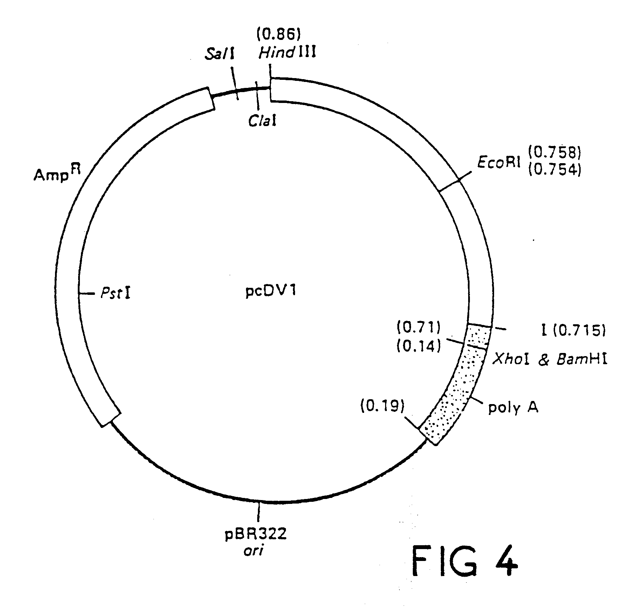

After amplification pL1' is purified, digested with HindIII and XhoI, and the small fragment containing the SV40 ori and R-U5' is isolated. Separately, plasmid pcDV1 (illustrated in Figure 4) is purified, digested with HindIII and XhoI, and the large fragment containing the pBR322 ori and Amp$^R$ gene is isolated. The small fragment of pL1' and the large fragment of pcDV1 are ligated, and the resultant plasmid pcD (SRα) is amplified. Separately plasmid pcD-human-GM-CSF is digested with XhoI and the small fragment containing the coding region for GM-CSF is isolated, and ligated with XhoI-digested pcD(SRα). The resulting recombinants molecules are transfected into E. coli, e.g. strain HB101 or MC1061, and plated. Bacteria from ampicillin-resistant colonies are randomly selected and separately amplified. Plasmids are extracted, purified, and then used to transfect COS7 monkey cells as described above. The COS7 cultures whose supernatants assay positive for GM-CSF activity carry the desired pcD (SRα)-hGM-CSF plasmids.

Example III. Expression of GM-CSF in Saccharomyces cerevisiae

The native signal peptide coding region of human GM-CSF was removed and replaced by the signal peptide coding region of the yeast α-factor mating pheromone in plasmid pMFα8. The yeast, Saccharomyces cerevisiae, was transformed by pMFα8, the mating factor/GM-CSF fusion protein was expressed, and mature GM-CSF was secreted into the culture medium. The results of this example are also described by Miyajima et al., in EMBO Journal, Vol. 5, pgs. 1193-1197 (1986).

S. cerevisiae secretes mating-type specific oligopeptide pheromones. MATα cells secrete α-factor, which induces the growth arrest of MATα cells at G1 phase of the cell cycle (Thorner, J., The Molecular Biology of the Yeast Saccharomyces, Cold Spring Harbor Laboratory, New York (1981); see particularly pages 143-180. The α-factor is initially synthesized as a larger precursor molecule consisting of an NH$_2$-terminal signal sequence of about 20 amino acids, followed by a leader sequence of an additional 60 amino acids, and ending with four identical tandem repeats of the mature α-factor sequence. The repeats are separated from each other by six or eight amino acids spacers (Lys-Arg-Glu-Ala-Glu-Ala and Lys-Arg-Glu-Ala-Glu[or Asp]-Ala-Glu-Ala). This prepro-α-factor is cleaved at several specific sites. The first processing is the cleavage of the COOH-terminal side of the Lys-Arg pair of the spacer sequence catalysed by the KEX2

14

product: Julius et al., Cell, Vol. 37, pgs. 1075-1089 (1984). A carboxypeptidase-B like enzyme cleaves at the NH₂-terminal side of the Lys-Arg pair. The final step is the removal of Glu-Ala or Asp-Ala pairs by diaminopeptidase, which is encoded by the STE13. J. Brake et al., Proc. Natl. Acad. Sci., Vol. 81, pgs. 4642-4646 (1984), have shown that the fusion of the sequence encoding mature human proteins to the first processing site allowed secretion of such proteins.

A general yeast expression vector, designated pMFα8, containing the alpha factor promoter and downstream leader sequence in conjunction with other elements, has been deposited with the ATCC (accession number 40140). It can be constructed as follows:

A 1.7 kb EcoRI fragment carrying the MFα1 gene (Kurjan, J. and Hershowitz, I., Cell, Vol. 30, pgs. 933-943 (1982) is cloned into the EcoRI restriction site of M13mp8 (Viera, J. and Messing, J., Gene, Vol. 19, pgs. 259-268 (1982). In order to introduce a HindIII site after the lysine codon of the first spacer region, the synthetic oligonucleotide TCTTTTATCCAAAGATACCC is hybridized to the single stranded M13-MFα1 DNA and the oligonucleotide primer extended by DNA polymerase I Klenow fragment. After S1 nuclease treatment, the DNA is cleaved with EcoRI and the fragment carrying the MFα1 promoter and leader sequence cloned into the EcoRI and filled-in HindIII restriction sites of pUC8 (Viera, J. and Messing, J., cited above). One plasmid with the desired structure can be isolated (designated pMFα4Δ1). The pMFα4Δ1 is cleaved with HindIII and partially filled in with DNA polymerase I Klenow fragment in the presence of dATP and dGTP. The DNA is treated with mung bean nuclease, and the oligonucleotide linker GCCTCGAGGC attached. The resultant plasmid (designated pMFα5) will have a StuI cleavage site immediately after an arginine codon, followed by the XhoI restriction site. An S. cerevisiae-E. coli shuttle vector (pTRP584) can be constructed as follows: the PstI-XbaI fragment carrying 2 μm plasmid replication origin (J. Broach, cited above), is cloned into the ClaI restriction site of pTRP56 (Miyajima et al., Mol. Cell. Biol., Vol. 4, pgs. 407-414 (1984)), and the StuI restriction site within the TRP1-ARS1 fragment converted to PvuII restriction site by PvuII linker insertion. The KpnI restriction site in the original pTRP56 is converted to XhoI by the XhoI linker insertion. The general secretion vector pMFα8 is then obtained by insertion of the BglII-XhoI fragment of pMFα5 into the BamHI-XhoI restriction sites of pTRP584.

Before the cloning into pMFα8, an FspI restriction site was introduced into the GM-CSF cDNA by oligonucleotide-directed mutagenesis in M13mp8 according to the procedure disclosed by Zoller and Smith in Methods in Enzymology, Vol. 100, pgs. 468-500 (1983). The introduced FspI site permits the cDNA insert to be cleaved at the junction between the endogenous signal peptide coding region and the region coding for the mature polypeptide prior to insertion into pMFα8.

Briefly, the BamHI fragment of pcD-human-GM-CSF containing the complete cDNA insert (see Figure 2A) was cloned into the replicative form of M13mp8, which was then used to transform E. coli JM101. M13mp8s carrying the insert were selected from clear plaques on agar plates containing isopropyl-beta-D-thiogalactosidase (IPTG) and 5-bromo-4-chloro-3-indolyl-beta-D-galactoside (X-gal). Single stranded M13mp8 DNA was separately prepared from eight E. coli JM101 cultures corresponding to selected plaques using standard techniques (centrifugation, removal of phage particles from supernatant with polyethylene glycol, phenol extraction to separate the DNA from the phage coats, and ethanol precipitation).

The eight sets of DNAs were grouped into two subsets according to the orientation of the cDNA insert, as determined by cross hybridization and gel electrophoresis. Oligonucleotide-directed mutagenesis was carried out separately on single stranded DNAs from each subset. Alternatively, members from each of the two subsets could have been sequenced to determine the single stranded DNAs containing the cDNA insert in the correct orientation.

A 20-mer oligonucleotide primer was synthesized (Applied Biosystems, Inc., model 380A, Foster City, CA) having the following sequence (the mismatches are underlined):


GTCGTAGACGCGTGGGCGGG


After 3-phosphorylation, the 20-mer oligonucleotide primer was mixed in approximately 30-fold excess with the single stranded DNA (approximately 1 μg), and heated to 65°C for 5 min in 0.5 M NaCl in a 5 μl volume and allowed to stand at room temperature for 1 hour. Buffer and salt concentrations were adjusted to 100mM NaCl, 20mM Tris•HCl at pH 7.5, 10mM dithiothreitol, 10mM MgCl₂, each of the four deoxynucleoside triphosphates to about 400μM, and ATP at about 500μM. DNA polymerase I (Klenow fragment) and T4 DNA ligase were then added and the reaction mixture was allowed to incubate at 12°C. Double stranded closed-loop DNAs were isolated by alkaline sucrose gradient centrifugation, and used to transform CaCl₂-treated E. coli JM101. E. coli JM101 colonies were screened for the presence of the mutant cDNA by

hybridization to a $^{32}$P-labeled oligonucleotide probe having the same sequence as that used for the primer.

After amplification, the mutant-carrying M13 phage was digested with FspI and BamHI, treated with DNA polymerase I Klenow fragment (to blunt the protruding end of the BamHI cut), and subjected to gel electrophoresis. Finally, the isolated GM-CSF-containing fragment was inserted into the StuI site of pMFα8.

S. cerevisiae 20B-12 (MATα trp1-289 pep 4-3) was transformed with the pMFα8 by the lithium acetate method (Ito et al., J. Bacteriol., Vol. 153, pgs. 163-168 (1983)) and grown in medium containing 0.67% yeast nitrogen base without amino acids, 0.5% casamino acids, and 2% glucose.

Colony assays with umbilical cord blood confirmed the presence of secreted GM-CSF in the yeast medium.

Example IV. Expression of GM-CSF in Escherichia coli

The coding region for mature human GM-CSF was inserted in the vector pIN-III-OmpA2 which contains the signal peptide coding sequence of the ompA protein. pIN-III-OmpA2 is disclosed by Ghrayeb et al. in EMBO Journal, Vol. 3, pgs. 2437-2442 (1984), and Masui et al. in Biotechnology, Vol. 2, pgs. 81-85 (1984). Accordingly, these references are incorporated by reference.

The insertion was carried out in three steps. First, the PstI/BamHI fragment of pcD-human-GM-CSF containing the mature GM-CSF coding region was inserted into PstI/BamHI-digested M13mp10 replicative form (RF), and an EcoRI site was introduced adjacent to, and 5,- of, the initial codon of the mature GM-CSF by site-directed mutagenesis. Secondly, the EcoRI/BamHI fragment of the mutated M13mp10 was inserted into EcoRI/BamHI-digested pIN-III-ompA2. Finally, a superfluous nine-base-pair sequence between the OmpA signal peptide coding region and the mature GM-CSF coding region in pIN-III-OmpA2 was removed by site-directed mutagenesis. In all cases, mutant forms of M13 were detected by using the synthetic primer as a probe.

All restriction enzyme digestions, DNA polymerase, kinase and ligase reactions were performed essentially as described by Maniatis et al. (cited above). Enzymes were purchased from New England Biolabs and Boehringer Mannheim. Plasmid isolations were done according to the alkaline method (small scale: Maniatis et al., cited above) or a modification of this method (large scale: Zurawski et al., J. Immunol., Vol. 137, pgs. 3354-3360 (1986). Site-directed mutagenesis was performed on DNA fragments subcloned into a M13mp10 vector. Ten ng of kinased primer DNA was mixed with 50-100 ng single stranded template DNA in ligase buffer in a volume of 40 $\mu$l. To this mix 100 ng of linearized M13 RF was added. The reaction mix was heated for 10 min. at 95°C. Annealing occurred at room temperature for 30 min. followed by 15 min. at 4°C. dNTPs (50 $\mu$M), ligase (400 U) and Klenow (5 U) were added (total volume 50 $\mu$l) and the mixture incubated for 30 min. at 4°C followed by 1 hr at 12°C. The mixture was transformed into JM101 cells and plated on L broth (LB) plates in the presence of IPTG and X-gal. White plaques were picked into microtiter dishes in 150 $\mu$l L broth. M13-infected cells were transferred to LB plates overlayed with a lawn of JM101 cells with the use of a stamp. Following incubation for 16 hrs at 37°C, pre-wet nitrocellulose paper was laid on plaques for 1 min. Filters were soaked in .05 M NaOH for 3 min, followed by 15 min in neutralization buffer (3 M NaCl, 0.5 M Tris-HCl pH7.5). After transfer to fresh neutralization buffer for another 15 min, the filters were submerged in 2xSSC. Filters were dried and baked at 80°C for 1.5 hrs. Pre-hybridization of filters was for 3 hrs at room temperature in 0.09M Tris-HCl pH7.5, 1x Denhardts, 0.9M NaCl, 0.1mM ATP, 1mM Pi, 1mM PP, 0.5% NP40, 6mM EDTA and 0.2 mg/ml E. coli tRNA. $^{32}$P labelled probe was added and incubation continued for 16 hrs at room temperature. Filters were washed in 6xSSC, 0.1% SDS, at increasing temperatures until background was washed off as monitored by autoradiography. If necessary the SSC concentration was lowered. Single stranded DNA from positive plaques was isolated and sequenced.

DNA sequence analysis was performed using standard dideoxy procedures (Sanger et al., Proc. Natl. Acad. Sci., Vol. 74, pgs. 5463-5467 (1977)). DNA oligonucleotides were synthesized by phosphoramidite chemistry using an Applied Biosystems 380A synthesizer.

The PstI/BamHI fragment of pcD-human-GM-CSF was cloned into PstI- and BamHI digested M13mp10 RF. Single stranded DNA of this construction was prepared using standard techniques (Messing, cited above), and site-directed mutagenesis was used to introduce an EcoRI site (GAATTC) between base pairs 83 and 84 of the GM-CSF cDNA sequence illustrated in Figure 1. The following synthetic primer was used:

5'-CTGCAGCATCTCTGAATTCGCACCCGCCCGCT-3'

The EcoRI site is indicated by underlining. After sequence confirmation of the insertion mutants, the EcoRI/BamHI fragment of M13 containing GM-CSF cDNA was inserted into EcoRI/BamHI digested pIN-III-OmpA2, which was amplified in E. coli JM101, isolated, and digested with XbaI and BamHI. The XbaI/BamHI fragment containing GM-CSF cDNA was inserted into XbaI/BamHI-digested M13mp10 RF. Single stranded DNA was isolated using standard techniques and site-directed mutagenesis was carried out using the following synthetic primer:

5'-GTAGCGCAGGCC GCACCCGCCCGCT-3'

GCTGAATTC

The gap in the primer sequence and the underlined sequence below indicate the location of the 9-base-pair deletion and the deleted sequence itself. After sequence confirmation of the deletion mutant, an XbaI/BamHI fragment thereof was isolated and ligated to XbaI/BamHI-digested pIN-III-OmpA2 to give the final construction, which was used to transform E. coli JM101.

Example V. Purification of GM-CSF Expressed in Escherichia coli

Purification of GM-CSF from a soluble E. coli extract was accomplished by sequential anion-exchange, dye-ligand affinity, gel filtration, and reverse-phase chromatographies. Preferably, the sequence of chromatographies consists of QAE (quaternary aminoethyl) column chromatography, Matrex Gel Red A column chromatography, concentration by ultrafiltration and/or ammonium sulfate precipitation, Sephadex G-100 gel filtration, and either FPLC or HPLC reverse-phase chromatography. The final product by this procedure demonstrated high specific biological activity, 95-100% electrophoretic purity, a low level of pyrogens, and a low level of either E. coli-derived contaminants or derivatives of GM-CSF.

All operations were performed at 2-15°C, unless otherwise indicated. Protein concentration was determined at each stage by a Coomassie blue binding assay. pH measurements may vary by + 0.2 pH units and conductivity measurements may vary by + 3mS. If the expected degree of purification was not achieved in any chromatographic procedure, eluted fractions were rechromatographed on the same column, or alternatively, recycled through a previous step or a previous series of steps. An ammonium sulfate precipitation and/or ultrafiltration step may be performed to concentrate and/or store the protein; as e.g., in step C. All solvents, including those that are employed for either equilibration or elution of columns, were filtered prior to use through a 0.2 $\mu$ membrane. USP XIX grade water was employed in the preparation of all solvents after step D.

A. Cell Killing And Protein Extractions

Cells were killed by adjusting the pH to 2.0 by first adding 85% phosphoric acid to bring the pH to 4.5 and then by adding 50% trichloroacetic acid to bring the pH to 2.0. After filtration through a 0.2 $\mu$ membrane, the conductivity was adjusted to 15-20 mS by addition of water. A low conductivity was required to minimize the amount of dilution required to allow GM-CSF to bind to the QAE column.

B. Quaternary Aminoethyl (QAE) Column Chromatography.

If required, the neutralized extract was adjusted to pH 7.5 with sodium hydroxide or hydrochloric acid, as appropriate. The conductivity of the neutralized extract was adjusted to 5-10 mS by addition of deionized water. The QAE column was equilibrated with at least 2-3 column volumes of 20 mM Tris-HCl, pH 7.5. The extract containing GM-CSF was applied to the column at a loading of not greater than 20 mg per ml of resin. The column was eluted with 10-20 column volumes of a gradient of sodium chloride in the range of 0-0.5 M dissolved in the same buffer in which the column was equilibrated. Fractions were combined based on sodium dodecyl sulfate-polyacrylamide gel electrophoresis (SDS-PAGE) and chromatographed in Step C.

C. Dye Affinity Chromatography

The combined fractions from the QAE column were diluted with deionized water to a conductivity of 5-10 mS and loaded onto a Red affinity column (e.g., Procion Red HE3B attached to an agarose support) that had been previously equilibrated with 2-3 column volumes of 20 mM Tris-HCl, pH 7.5. The amount of protein loaded onto the column should not exceed 10 mg per ml of gel. The column was washed with 3-4 column volumes of the equilibration buffer. Elution was performed with between 5 and 15 volumes of a gradient of sodium chloride from 0 to 0.75 M dissolved in 20 mM Tris-HCl, pH 7.5. The fractions to be employed in Step D are combined on the basis of SDS-PAGE.

D. Concentration By Ultrafiltration and/or Ammonium Sulfate Precipitation

If the protein concentration of the combined fractions from the previous stage was less than 1.0 mg/ml, the solution was concentrated by ultrafiltration employing a 3,000-5,000 molecular weight cut-off membrane. The final protein concentration should be 1.0 mg/ml or greater. Ammonium sulfate was added to a final concentration of 60-70%. The precipitate was collected by centrifugation and then washed once with 20 mM Tris-HCl (pH 7.5) containing the same concentration of ammonium sulfate employed for precipitation. The precipitate was collected by centrifugation and dissolved in 20 mM Tris-HCl, pH 7.5.

E. Sephadex G-100 Column Chromatography

The redissolved ammonium sulfate precipitate was clarified by centrifugation, if necessary. The solution was filtered through a 0.2 μ filter and charged onto a Sephadex G-100 column equilibrated with 20 mM Tris-HCl, pH 7.5. The amount of protein loaded onto the column should not exceed 3 mg per ml of gel. The column was eluted with the same buffer. Fractions for Step F were combined based on SDS-PAGE.

F. Reversed-Phase Column Chromatography

The combined Sephadex G-100 fractions were passed through a 0.2 μ filter and applied to a reversed-phase column; as, e.g., FPLC (fast protein liquid chromatography) and HPLC (high performance liquid chromatography) columns. The column was equilibrated with 0.1% trifluoroacetic acid (TFA) and elution was performed with a gradient of 0-100% acetonitrile dissolved in 0.1% TFA. Fractions were combined based on SDS-PAGE. The solution was lyophilized and the dried powder was dissolved in 20 mM sodium phosphate, pH 7.2.

G. Dialysis Of The Purified GM-CSF

The solution from Step F was dialyzed against 20 mM sodium phosphate, pH 7.2. Two changes of buffer were performed with a minimum of 4-5 hours between changes. If required, the dialyzed solution was concentrated by ultrafiltration using a 3,000-5,000 molecular weight cut-off membrane to a protein concentration of at least 1.0 mg/ml. The solution of purified GM-CSF was passed through a 0.2 μ filter and stored frozen at about -20°C or below.

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to best utilize the invention in various embodiments and with various modifications as are suited to the particular use contemplated. It is intended that the scope of the invention be defined by the claims appended hereto.

Applicants have deposited pcD-human-GM-CSF with the American Type Culture Collection, Rockville, MD, USA (ATCC), under accession number 39923. This deposit satisfies the requirements of the Budapest Treaty.

**Claims**

1. An expression vector for the production of human granulocyte-macrophage colony stimulating factor in a mammalian cell host, the expression vector comprising in sequence:
   an SV40 origin of DNA replication;

an SV40 early region promoter;

a promoter;

a splice junction and a nucleotide sequence capable of encoding human granulocyte-macrophage colony stimulating factor; and

a polyadenylation site;

characterized in that the promoter is the SRα promoter of the plasmid deposited in ATCC 67318.

2. The expression vector of claim 1, characterized in that it further includes in sequence after said SV40 polyadenylation site:

a bacterial origin of replication permitting said expression vector to be cloned in a bacterial host; and

a selectable marker permitting the identification of bacterial hosts transformed by said expression vector.

3. The expression vector of claim 2, characterized in that said bacterial origin of replication is the pBR322 origin of replication, said bacterial host is Escherichia coli, and said selectable marker confers antibiotic resistance to Escherichia coli.

4. The expression vector of claim 3, characterized in that said mammalian cell host is selected from COS monkey cells, CV1 monkey cells, mouse L cells, and Chinese hamster ovary cells.

5. A process for the production of human granulocyte-macrophage colony stimulating factor in a mammalian cell host, characterized in that an expression vector as claimed in any of claims 1 to 3 is cultivated in a suitable mammalian cell host.

6. A process as claimed in claim 5, characterized in that said mammalian cell host is selected from COS monkey cells, CV1 monkey cells, mouse L cells, and Chinese hamster ovary cells.

**Patentansprüche**

1. Expressionsvektor zur Herstellung eines das Wachstum einer Human-Granulozyt-Makrophagenkolonie stimulierenden Faktors in einem Säugerzellen-Wirt, wobei der Expressionsvektor die Sequenz

einen SV40-DNS-Replikationsstartpunkt,

einen SV40-Frühregion-Promoter,

einen Promoter

eine Spleißverbindung und eine Nukleotidsequenz, welche befähigt ist, einen das Wachstum einer Human-Granulozyt-Makrophagenkolonie stimulierenden Faktor zu kodieren und

eine Polyadenylierungsstelle

enthält, dadurch gekennzeichnet, daß der Promoter der SR -Promoter des als ATTCC 67318 hinterlegten Plasmids ist.

2. Expressionsvektor des Anspruchs 1, dadurch gekennzeichnet, daß er weiterhin nach der SV40-Polyadenylierungsstelle der Reihe nach

einen bakteriellen Replikationsstartpunkt, welcher es erlaubt, den Expressionsvektor in einem bakteriellen Wirt zu klonen, und

einen auswählbaren Marker, welcher die Identifizierung von durch den Expressionsvektor transformierten bakterieilen Wirten erlaubt,

umfaßt.

3. Expressionsvektor des Anspruchs 2, dadurch gekennzeichnet, daß der bakterielle Replikationsstartpunkt der pBR322-Replikationsstartpunkt ist, der bakterielle Wirt Escherichia coli ist, und der auswählbare Marker Escherichia coli Antibiotikaresistenz verleiht.

4. Expressionsvektor des Anspruchs 3, dadurch gekennzeichnet, daß der Säugerzellen-Wirt aus COS-Affenzellen, CV1-Affenzellen, Maus-L-Zellen und Eizellen des chinesischen Hamsters ausgewählt ist.

5. Verfahren zur Herstellung eines das Wachstum einer Human-Granulozyt-Makrophagenkolonie stimulierenden Faktors in einem Säugerzellen-Wirt, dadurch gekennzeichnet, daß ein wie in einem der Ansprüche 1 bis 3 beanspruchter Expressionsvektor in einem geeigneten Säugerzellen-Wirt kultiviert wird.

**6.** Verfahren wie in Anspruch 5 beansprucht, dadurch gekennzeichnet, daß der Säugerzellen-Wirt aus COS-Affenzellen, CV1-Affenzellen, Maus-L-Zellen und Eizellen des chinesischen Hamsters ausgewählt ist.

**Revendications**

**1.** Vecteur d'expression pour la production d'un facteur de stimulation des colonies de granulocytes-macrophages humains dans une cellule hôte mammifère, le vecteur d'expression comprenant en séquence :

une origine de réplication d'ADN de SV40 ;

un promoteur de la région précoce de SV40 ;

un promoteur ;

une jonction d'épissure et une séquence de nucléotides permettant le codage d'un facteur de stimulation de colonies de granulocytes-macrophages humains ; et

un site de polyadénylation ;

caractérisé en ce que le promoteur est le promoteur SRα du plasmide déposé sous le N° ATCC 67318.

**2.** Vecteur d'expression de la revendication 1, caractérisé en ce qu'il contient de plus en séquence, après ledit site de polyadénylation de SV40 :

une origine bactérienne de réplication permettant audit vecteur d'expression d'être cloné chez un hôte bactérien ; et

un marqueur sélectionnable permettant l'identification des hôtes bactériens transformés par ledit vecteur d'expression.

**3.** Vecteur d'expression de la revendication 2, caractérisé en ce que ladite origine bactérienne de réplication est l'origine de réplication de pBR322, ledit hôte bactérien est Escherichia coli et ledit marqueur sélectionnable confère une résistance aux antibiotiques à Escherichia coli.

**4.** Vecteur d'expression de la revendication 3, caractérisé en ce que ladite cellule hôte mammifère est choisie parmi les cellules de singe COS, les cellules de singe CV1, les cellules L de souris et les cellules d'ovaire de hamster chinois.

**5.** Procédé de production d'un facteur de stimulation de colonies de granulocytes-macrophages humains dans une cellule hôte mammifère, caractérisé en ce qu'on cultive un vecteur d'expression selon l'une quelconque des revendications 1 à 3 dans une cellule hôte mammifère appropriée.

**6.** Procédé selon la revendication 5, caractérisé en ce que ladite cellule hôte mammifère est choisie parmi les cellules de singe COS, les cellules de singe CV1, les cellules L de souris et les cellules d'ovaire de hamster chinois.

```
             10          20          30                      47
ACACAGAGAG AAAGGCTAAA GTTCTCTGGA GG ATG TGG CTG CAG AGC CTG CTG CTC TTG
                                    MET Trp Leu Gln Ser Leu Leu Leu Leu

    62                      77                      92                 107
GGC ACT GTG GCC TGC AGC ATC TCT GCA CCC GCC CGC TCG CCC AGC CCC AGC ACG
Gly Thr Val Ala Cys Ser Ile Ser Ala Pro Ala Arg Ser Pro Ser Pro Ser Thr

            122                 137                 152                 167
CAG CCC TGG GAG CAT GTG AAT GCC ATC CAG GAG GCC CGG CGT CTC CTG AAC CTG
Gln Pro Trp Glu His Val Asn Ala Ile Gln Glu Ala Arg Arg Leu Leu Asn Leu

                182                 197                 212
AGT AGA GAC ACT GCT GCT GAG ATG AAT GAA ACA GTA GAA GTC ATC TCA GAA ATG
Ser Arg Asp Thr Ala Ala Glu Met Asn Glu Thr Val Glu Val Ile Ser Glu Met

    227                 242                 257                 272
TTT GAC CTC CAG GAG CCG ACC TGC CTA CAG ACC CGC CTG GAG CTG TAC AAG CAG
Phe Asp Leu Gln Glu Pro Thr Cys Leu Gln Thr Arg Leu Glu Leu Tyr Lys Gln

                287                 302                 317
GGC CTG CGG GGC AGC CTC ACC AAG CTC AAG GGC CCC TTG ACC ATG ATG GCC AGC
Gly Leu Arg Gly Ser Leu Thr Lys Leu Lys Gly Pro Leu Thr Met Met Ala Ser

332                 347                 362                 377
CAC TAC AAG CAG CAC TGC CCT CCA ACC CCG GAA ACT TCC TGT GCA ACC CAG ATT
His Tyr Lys Gln His Cys Pro Pro Thr Pro Glu Thr Ser Cys Ala Thr Gln Ile

            392                 407                 422                 437
ATC ACC TTT GAA AGT TTC AAA GAG AAC CTG AAG GAC TTT CTG CTT GTC ATC CCC
Ile Thr Phe Glu Ser Phe Lys Glu Asn Leu Lys Asp Phe Leu Leu Val Ile Pro

                452                 467       477       487       497
TTT GAC TGC TGG GAG CCA GTC CAG GAG TGA GACCGGCCAG ATGAGGCTGG CCAAGCCGGG
Phe Asp Cys Trp Glu Pro Val Gln Glu  .

        507       517       527       537       547       557
GAGCTGCTCT CTCATGAAAC AAGAGCTAGA AACTCAGGAT GGTCATCTTG GAGGGACCAA

        567       577       587       597       607       617
GGGGTGGGCC ACAGCCATGG TGGGAGTGGC CAGGACCTGC CCTGGGCACA CTGACCCTGA

        627       637       647       657       667       677
TACAGGCATG GCAGAAGAAT GGGAATATTT TATACTGACA GAAATCAGTA ATATTTATAT

        687       697       707       717       727       737
ATTTATATTT TTAAAATATT TATTTATTTA TTTATTTAAG TTCATATTCC ATATTTATTC

        747       757       767       777       787
AAGATGTTTT ACCGTAATAA TTATTATTAA AAATATGCTT CTAAAAAAAA
```

FIG 1

FIG 2 A

FIG 2 B

FIG 3

FIG 4